# EUROPEAN PATENT SPECIFICATION

(11) **EP 2 274 016 B1**
(45) Date of publication and mention of the grant of the patent: **25.07.2012**
(21) Application number: 09727511.9
(22) Date of filing: 24.03.2009
(51) Int. Cl.: A61K 47/48, A61P 25/00

(54) **PEGYLATED IGF-I VARIANTS FOR USE IN THE TREATMENT OF NEUROMUSCULAR DISORDERS**
PEGYLIERTE IGF-I-VARIANTEN ZUR VERWENDUNG BEI DER BEHANDLUNG NEUROMUSKULÄRER ERKRANKUNGEN
VARIANTES DE L'IGF-I PEGYLÉES POUR UTILISATION DANS LE TRAITEMENT DES TROUBLES NEUROMUSCULAIRES

(30) Priority: 03.04.2008 EP 08153994
(43) Date of publication of application: 19.01.2011
(73) Proprietor: F. Hoffmann-La Roche AG, 4070 Basel (CH)
(72) Inventor: HOLTMANN, Bettina, 97080 Wuerzburg (DE); METZGER, Friedrich, 79115 Freiburg (DE); SENDTNER, Michael, 97209 Veitshoechheim (DE)
(74) Representative: Küng, Peter
(86) International application number: PCT/EP2009/053465
(87) International publication number: WO 2009/121759

(56) References cited:
- WO-A1-95/32003
- WO-A1-2008/025528
- WO-A2-2006/066891
- WO-A2-2007/084743
- LAI E C ET AL: "Effect of recombinant human insulin-like growth factor-I on progression of ALS: A placebo-controlled study" NEUROLOGY 199712 US, vol. 49, no. 6, December 1997 (1997-12), pages 1621-1630, XP8116424 ISSN: 0028-3878 cited in the application
- IWASAKI Y ET AL: "Prevention by insulin-like growth factor-I and riluzole in motor neuron death after neonatal axotomy" JOURNAL OF THE NEUROLOGICAL SCIENCES 19991031 NL, vol. 169, no. 1-2, 31 October 1999 (1999-10-31), pages 148-155, XP002560768 ISSN: 0022-510X
- BORASIO G D ET AL: "A placebo-controlled trial of insulin-like growth factor-I in amyotrophic lateral sclerosis" NEUROLOGY 199808 US, vol. 51, no. 2, August 1998 (1998-08), pages 583-586, XP8116423 ISSN: 0028-3878
- LAI E C: "Therapeutic developments in amyotrophic lateral sclerosis" EXPERT OPINION ON INVESTIGATIONAL DRUGS 1999 GB, vol. 8, no. 4, 1999, pages 347-361, XP002560770 ISSN: 1354-3784

### SEQUENCE LISTING

<110> F. Hoffmann-La Roche AG
<120> Use of PEGylated IGF-I variants for the treatment of neuromuscular disorders
<130> 24831
<160> 4
<170> PatentIn version 3.4
<210> 1
   <211> 70
   <212> PRT
   <213> Homo sapiens
<400> 1
   Gly Pro Glu Thr Leu Cys Gly Ala Glu Leu Val Asp Ala Leu Gln Phe
   1               5                   10                  15
   Val Cys Gly Asp Arg Gly Phe Tyr Phe Asn Lys Pro Thr Gly Tyr Gly
               20                  25                  30
   Ser Ser Ser Arg Arg Ala Pro Gln Thr Gly Ile Val Asp Glu Cys Cys
           35                  40                  45
   Phe Arg Ser Cys Asp Leu Arg Arg Leu Glu Met Tyr Cys Ala Pro Leu
       50                  55                  60
   Lys Pro Ala Lys Ser Ala
   65                  70
<210> 2
   <211> 70
   <212> PRT
   <213> Homo sapiens
<220>
   <221> MUTAGEN
   <222> (65)..(65)
   <223> K65R
<220>
   <221> MUTAGEN
   <222> (68)..(68)
   <223> K68R
<400> 2
   Gly Pro Glu Thr Leu Cys Gly Ala Glu Leu Val Asp Ala Leu Gln Phe
   1               5                   10                  15
   Val Cys Gly Asp Arg Gly Phe Tyr Phe Asn Lys Pro Thr Gly Tyr Gly
               20                  25                  30
   Ser Ser Ser Arg Arg Ala Pro Gln Thr Gly Ile Val Asp Glu Cys Cys
           35                  40                  45
   Phe Arg Ser Cys Asp Leu Arg Arg Leu Glu Met Tyr Cys Ala Pro Leu
       50                  55                  60
   Arg Pro Ala Arg Ser Ala
   65                  70
<210> 3
   <211> 70
   <212> PRT
   <213> Homo sapiens
<220>
   <221> MUTAGEN
   <222> (27) .. (27)
   <223> K27R
<220>
   <221> MUTAGEN
   <222> (68)..(68)
   <223> K68R
<400> 3
   Gly Pro Glu Thr Leu Cys Gly Ala Glu Leu Val Asp Ala Leu Gln Phe
   1               5                   10                  15
   Val Cys Gly Asp Arg Gly Phe Tyr Phe Asn Arg Pro Thr Gly Tyr Gly
               20                  25                  30
   Ser Ser Ser Arg Arg Ala Pro Gln Thr Gly Ile Val Asp Glu Cys Cys
           35                  40                  45
   Phe Arg Ser Cys Asp Leu Arg Arg Leu Glu Met Tyr Cys Ala Pro Leu
       50                  55                  60
   Lys Pro Ala Arg Ser Ala
   65                  70
<210> 4
   <211> 70
   <212> PRT
   <213> Homo sapiens
<220>
   <221> MUTAGEN
   <222> (27)..(27)
   <223> K27R
<220>
   <221> MUTAGEN
   <222> (68)..(68)
   <223> K68R
<400> 4
   Gly Pro Glu Thr Leu Cys Gly Ala Glu Leu Val Asp Ala Leu Gln Phe
   1               5                   10                  15
   Val Cys Gly Asp Arg Gly Phe Tyr Phe Asn Arg Pro Thr Gly Tyr Gly
               20                  25                  30
   Ser Ser Ser Arg Arg Ala Pro Gln Thr Gly Ile Val Asp Glu Cys Cys
           35                  40                  45
   Phe Arg Ser Cys Asp Leu Arg Arg Leu Glu Met Tyr Cys Ala Pro Leu
       50                  55                  60
   Arg Pro Ala Lys Ser Ala
   65                  70

## Description

The present invention relates to polyethylene glycol-(PEG)ylated IGF-I variants for use in the treatment, prevention and/or delay of progression of neuromuscular disorders according to the claims, in particular amyotrophic lateral sclerosis (ALS). More specifically, the present invention relates to the use of a PEGylated IGF-I variant for the manufacture of a pharmaceutical composition for the treatment, prevention and/or delay of neuromuscular disorders according to the claims, in particular ALS wherein the PEGylated IGF-I variant is characterized in that it is derived from the wild-type human IGF-I amino acid sequence (SEQ ID NO: 1) and carries one or two amino acid alterations at amino acid positions 27, 65 and 68 so that one or two of amino acids at positions 27, 65 and 68 is/are a polar amino acid but not lysine and PEG is attached to at least one lysine residue.

Neuromuscular disorders cover a range of conditions including neuropathies (either acquired or inherited), muscular dystrophies, ALS, spinal muscular atrophy (SMA), as well as a range of very rare muscle disorders. Neuromuscular disorders affect the nerves that control voluntary muscles. When the neurons become unhealthy or die, communication between the nervous system and muscles breaks down. As a result, muscles weaken and waste away. The weakness can lead to twitching, cramps, aches and pains, and joint and movement problems. Sometimes it also affects heart function and your ability to breathe. There are many causes of progressive muscle weakness, which can strike any time from infancy through adulthood.

Muscular dystrophy (MD) is a subgroup of neuromuscular disorders. MD represents a family of inherited diseases of the muscles. Some forms affect children (e.g., Duchenne dystrophy) and are lethal within two to three decades. Other forms present in adult life and are more slowly progressive. The genes for several dystrophies have been identified, including Duchenne dystrophy (caused by mutations in the dystrophin gene) and the teenage and adult onset Miyoshi dystrophy or its variant, limb girdle dystrophy 2B or LGMD-2B (caused by mutations in the dysferlin gene). These are "loss of function" mutations that prevent expression of the relevant protein in muscle and thereby cause muscle dysfunction. Mouse models for these mutations exist, either arising spontaneously in nature or generated by inactivation or deletion of the relevant genes. These models are useful for testing therapies that might replace the missing protein in muscle and restore normal muscle function.

Neuromuscular disorders also include motor neuron diseases (MND) which belong to a group of neurological disorders attributed to the destruction of motor neurons of the central nervous system and degenerative changes in the motor neuron pathway, and are different from other neurodegenerative diseases, such as Parkinson's disease, Alzheimer's disease, olivopontocerebellar atrophy, etc., which are caused by the destruction of neurons other than motor neurons. The National Institute of Neurological Diseases and Stroke (NINDS) calls motor neuron diseases (MNDs) progressive, degenerative disorders that affect nerves in the upper or lower parts of the body. Some are inherited, according to NINDS. Generally, MNDs strike in middle age. Symptoms may include difficulty swallowing, limb weakness, slurred speech, impaired gait, facial weakness and muscle cramps. Respiration may be affected in the later stages of these diseases. The cause(s) of most MNDs are not known, but environmental, toxic, viral or genetic factors are all suspects. Forms of MND include Adult Spinal Muscular Atrophy (SMA), Amyotrophic Lateral Sclerosis (ALS) which is also known as Lou Gehrig's Disease, Infantile Progressive Spinal Muscular Atrophy (SMA1) which is also known as SMA Type 1 or Werdnig-Hoffman, Intermediate Spinal Muscular Atrophy (SMA2) which is also known as SMA Type 2, Juvenile Spinal Muscular Atrophy (SMA3) which is also known as SMA Type 3 or Kugelberg-Welander, Spinal Bulbar Muscular Atrophy (SBMA) which is also known as Kennedy's Disease or X-linked SBMA. Motor neuron diseases are disorders in which motor neurons degenerate and die. Motor neurons, including upper motor neurons and lower motor neurons, affect voluntary muscles, stimulating them to contract. Upper motor neurons originate in the cerebral cortex and send fibers through the brainstem and the spinal cord, and are involved in controlling lower motor neurons. Lower motor neurons are located in the brainstem and the spinal cord and send fibers out to muscles. Lower motor neuron diseases are diseases involving lower motor neuron degeneration. When a lower motor neuron degenerates, the muscle fibers it normally activates become disconnected and do not contract, causing muscle weakness and diminished reflexes. Loss of either type of neurons results in weakness, muscle atrophy (wasting) and painless weakness are the clinical hallmarks of MND.

ALS is a fatal motor neuron disease characterized by the selective and progressive loss of motor neurons in the spinal cord, brainstem and cerebral cortex. It typically leads to progressive muscle weakness and neuromuscular respiratory failure. Approximately 10% of ALS are associated with point mutations in the gene coding for the Cu/Zn superoxide dismutase-1 enzyme (SOD1). The discovery of this primary genetic cause of ALS has provided a basis for testing various therapeutic possibilities. The potent neuroprotective activities of neurotrophic factors (NTFs), ranging from prevention of neuronal atrophy, axonal degeneration and cell death, generated a great deal of hope for the treatment of ALS in the early 90s. Ciliary neurotrophic factor (CNTF), brain-derived neurotrophic factor (BDNF) and insulin-like growth factor 1 (IGF-1) have already been evaluated in ALS patients. The rationale for testing these factors in ALS patients was based on their trophic effects on naturally occurring cell death paradigms during development, traumatic nerve injury or in animal models resembling ALS such as pmn or wobbler mice. Except IGF-1 (Lai EC et al. Neurology 1997, 49: 1621-1630), systemic delivery of these recombinant proteins did not lead to clinically beneficial effects in ALS patients (Turner MR at al. Semin. Neurol. 2001; 21: 167-175). Undesirable side effects and limited bioavailability have complicated the evaluation of their potential clinical benefits. A practical difficulty in applying neurotrophins is that these proteins all have a relatively short half life while the neurodegenerative diseases are chronic and require long term treatment.

Contemporaneously, several strains of transgenic mice overexpressing different ALS-linked SOD1 mutations have been generated (Newbery HJ et al. Trends Genet. 2001; 17: S2-S6). By closely mimicking many of the clinical and neuropathological features of ALS, these mice have provided more relevant animal models for investigating the preclinical potential of neurotrophic factors. Direct administration of recombinant trophic proteins has been disappointing. Beneficial effects on motor neuron neuropathology are subtle or null (Azari MF et al. Brain Res. 2003; 982: 92-97; Feeney SJ et al. Cytokine 2003; 23: 108-118; Dreibelbis JE et al. Muscle Nerve 2002; 25: 122-123). Viral vector-mediated delivery of neurotrophic factors such as glial cell line-derived neurotrophic factor (GDNF), IGF-I or cardiotrophin-1 (CT-1), however, revealed behavioral or neuropathological improvement (Wang LJ et al. J. Neurosci. 2002; 22: 6920-6928; Bordet T et al. Hum. Mol. Genet. 2001; 10: 1925-1933 and Kaspar BK et al. Science 2003, 301: 839-842) suggesting that with an appropriate application regimen efficacy can be achieved.

Insulin-like growth factor (IGF-I) is a circulating anabolic hormone structurally related to insulin. In the circulation, more than 99 % of IGF-I is bound to IGF-I binding proteins (IGFBP's), which have very high affinities to IGF's and modulate IGF-I function. The factor can be locally released from IGFBP's by proteolysis through specific proteases. The major source of serum IGF-1 (~75 %) is the liver (Sjögren, K., et al., Proc. Natl. Acad. Sci. 94 (1999) 7088-7092; Yakar, S., et al., Proc. Natl. Acad. Sci. 96 (1999) 7324-7329) although IGF-I is locally produced in every cell of the body. Besides its endocrine function, IGF-I has a paracrine role in the developing and mature brain (Werther, G.A., et al., Mol. Endocrinol. 4 (1990) 773-778). In vitro studies indicate that IGF-I is a potent non-selective trophic agent for several types of neurons in the CNS (Knusel, B., et al., J. Neurosci. 10 (1990) 558-570; Svrzic, D., and Schubert, D., Biochem. Biophys. Res. Commun. 172 (1990) 54-60), including dopaminergic neurons (Knusel, B., et al., J. Neurosci. 10(1990) 558-570), oligodendrocytes (McMorris, F.A., and Dubois-Dalcq, M., J. Neurosci. Res. 21 (1988) 199-209; McMorris, F.A., et al., Proc. Natl. Acad. Sci. USA 83 (1986) 822-826; Mozell, R.L., and McMorris, F.A., J. Neurosci. Res. 30 (1991) 382-390) and spinal motoneurons (Hughes, R.A., et al., J. Neurosci. Res. 36 (1993) 663-671; Neff, N.T., et al., J. Neurobiol. 24 (1993) 1578-1588; Li, L., et al., J. Neurobiol. 25 (1994) 759-766). The entrance of peripheral IGF-I into the brain through receptor-mediated transport across the blood-brain barrier (BBB) has been demonstrated (Rosenfeld, R.G. et al., Biochem. Biophys. Res. Commun. 149 (1987) 159-166; Duffy, K.R., et al., Metab. Clin. Exp. 37 (1988) 136-140; Pan, W. and Kastin, A.J.. Neuroendocrinology 72 (2000) 171-178). Preclinical data generated mainly in SOD1 transgenic mice provide strong evidence that IGF-I shows efficacy on ALS-related parameters when delivered either intrathecally or via slow release devices or gene therapeutic approaches (Kaspar et al., Science 301:839, 2003; Boillee and Cleveland, Trends Neurosci 27:235, 2004; Dobrowolny et al., J Cell Biol168:193, 2005; Nagano et al., J Neurol Sci 235:61, 2005; Narai et al., J Neurosci Res 82:452, 2005). This suggests that a constant delivery of IGF-I is required as no published data exist for efficacy in ALS models upon parenteral application of IGF-I doses suitable for use in humans.

US Patent No. 5,093,317 mentions that the survival of cholinergic neuronal cells is enhanced by administration of IGF-I. It is further known that IGF-I stimulate peripheral nerve regeneration (Kanje, M., et al., Brain Res. 486 (1989) 396-398) and enhance ornithine decarboxylase activity (US Patent No. 5,093,317). US Patent No. 5,861,373 and WO 93/02695 A1 mention a method of treating injuries to or diseases of the central nervous system that predominantly affects glia and/or non-cholinergic neuronal cells by increasing the active concentration(s) of IGF-I and/or analogues thereof in the central nervous system of the patient. WO 02/32449 A1 is directed to methods for reducing or preventing ischemic damage in the central nervous system of a mammal by administering to the nasal cavity of the mammal a pharmaceutical composition comprising a therapeutically effective amount of IGF-I or biologically active variant thereof. The IGF-I or variant thereof is absorbed through the nasal cavity and transported into the central nervous system of the mammal in an amount effective to reduce or prevent ischemic damage associated with an ischemic event. EP 0 874 641 A1 claims the use of an IGF-I or an IGF-II for the manufacture of a medicament for treating or preventing neuronal damage in the central nervous system, due to AIDS-related dementia, AD, Parkinson's Disease, Pick's Disease, Huntington's Disease, hepatic encephalopathy, cortical- basal ganglionic syndromes, progressive dementia, familial dementia with spastic parapavresis, progressive supranuclear palsy, multiple sclerosis, cerebral sclerosis of Schilder or acute necrotizing hemorrhagic encephalomyelitis, wherein the medicament is in a form for parenteral administration of an effective amount of said IGF outside the blood-brain barrier or blood- spinal cord barrier.

For clinical use, however, short half-life of IGF-I in the periphery after exogenous application is a clear disadvantage and requires high dosing frequency which generates severe issues. Side effects (as hypoglycaemia, seen frequently in clinical trials with IGF-I, also see NDA report 21-839 (http://www.fda.gov/cder/foi/nda/2005/021839 S000 Increlex Pharm.pdf) due to acute overload with IGF-I limit the maximum tolerated dose to a level where sustained efficacy is not yet reached. To overcome this disadvantage and achieve higher doses for better activity, a modified IGF-I with slower absorption rate, longer and stable blood residence but maintained bioactivity would be required. In order to ensure that said modified IGF-I can still exert its neuroprotective action, it is also required that the blood-brain barrier transport is fully working.

In preclinical use it has been tried to address at least some of the aforementioned difficulties by encapsulation of IGF-I into slow release devices as minipumps and microspheres for constant supply without large compound fluctuation in the blood (Carrascosa C et al. Biomaterials 25; 707-714; WO 03/077940 A1). However, using this approach an initial strong increase of blood IGF-I has been observed which will generate the same acute side effects in humans as s.c. injection of IGF-I.

Surprisingly, it has been found that specifically PEGylated IGF-I (PEG-IGF-I) variants when injected parenterally have the required pharmacokinetic profile. Said PEGylated IGF-I variants have no acute hypoglycaemic activity up to doses and/orplasma concentrations >10-fold higher than nonPEGylated IGF-I. One would have clearly expected that PEGylation impairs binding and receptor-mediated blood brain barrier penetration of IGF-I. The PEGylated IGF-I variants described hereinafter in detail are surprisingly neuroprotective and functional in animal, i.e. mouse models of neuromuscular disorders at much lower doses used than required with unPEGylated IGF-I, showing 1) that blood-brain barrier transport is fully working, 2) that the molecule fully maintains its biological activity in vivo and 3) that hypoglycaemia is seen only at >10-fold higher doses of PEG-IGF-I compared to IGF-I which allows even higher dosing of PEG-IGF-I for better efficacy in man.

### Summary of the invention

In a first embodiment the present invention relates to a pharmaceutically effective amount of a PEGylated IGF-I variant according to the claims for use in a method for the treatment of neuromuscular disorders according to the claims in a patient in need thereof

In a preferred embodiment the present invention relates to a pharmaceutically effective amount of a PEGylated IGF-I variant according to the claims for use in a method for the treatment of MND, in particular ALS in a patient in need thereof.

In another embodiment the present invention further relates to a pharmaceutical composition comprising a PEGylated IGF-I variant according to the claims together with a pharmaceutically acceptable carrier for use in a method for treatment, prevention and/or delay of progression of neuromuscular disorders, preferably MND, and even more preferably ALS.

A further aspect of the invention relates to the use of a PEGylated IGF-I variant according to the claims for the manufacture of a medicament for the treatment of neuromuscular disorders according to the claims, preferably MND, and even more preferably ALS.

### Detailed Description of the Invention

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods and materials are described hereinafter.

### Definitions

The term "neuromuscular disorders" encompasses diseases and ailments that either directly (via intrinsic muscle pathology) or indirectly (via nerve pathology) impair the functioning of muscle. Examples of neuromuscular disorders include.

Motor Neuron Diseases like ALS (also known as Lou Gehrig's Disease), Spinal Muscular Atrophy Type 1 (SMA1, Werdnig-Hoffmann Disease), Spinal Muscular Atrophy Type 2 (SMA2), Spinal Muscular Atrophy Type 3 (SMA3, Kugelberg-Welander Disease), Spinal Bulbar Muscular Atrophy (SBMA, also known as Kennedy Disease and X-Linked SBMA),

Muscular Dystrophies like Duchenne Muscular Dystrophy (DMD, also known as Pseudohypertrophic), Becker Muscular Dystrophy (BMD), Emery-Dreifuss Muscular Dystrophy (EDMD), Limb-Girdle Muscular Dystrophy (LGMD), Facioscapulohumeral Muscular Dystrophy (FSH or FSHD, also known as Landouzy-Dejerine), Myotonic Dystrophy (MMD, also known as Steinert Disease), Oculopharyngeal Muscular Dystrophy (OPMD), Distal Muscular Dystrophy (DD, Miyoshi), Congenital Muscular Dystrophy (CMD),

Metabolic diseases of muscle like Phosphorylase Deficiency (MPD or PYGM, also known as McArdle Disease), Acid Maltase Deficiency (AMD, also known as Pompe Disease), Phosphofructokinase Deficiency (also known as Tarui Disease), Debrancher Enzyme Deficiency (DBD, also known as Cori or Forbes Disease), Mitochondrial Myopathy (MITO), Carnitine Deficiency (CD), Carnitine Palmityl Transferase Deficiency (CPT), Phosphoglycerate Kinase Deficiency, Phosphoglycerate Mutase Deficiency, Lactate Dehydrogenase Deficiency, Myoadenylate Deaminase Deficiency ne Palmityl Transferase Deficiency (CPT), Phosphoglycerate Kinase Deficiency, Phosphoglycerate Mutase Deficiency, Lactate Dehydrogenase Deficiency, Myoadenylate Deaminase Deficiency;

Diseases of peripheral nerve like Charcot-Marie-Tooth Disease (CMT, also known as Hereditary Motor and Sensory Neuropathy (HMSN) or Peroneal Muscular Atrophy (PMA), Friedreich's Ataxia (FA), Dejerine-Sottas Disease (DS),

Inflammatory myopathies like Dermatomyositis (DM), Polymyositis (PM), Inclusion Body Myositis (IBM),

Diseases of the neuromuscular junction like Myasthenia Gravis (MG), Lambert-Eaton Syndrome (LES), Congenital Myasthenic Syndrome (CMS),

Myopathies due endocrine abnormalities like Hyperthyroid Myopathy (HYPTM), Hypothyroid Myopathy (HYPOTM)

Other myopathies like Myotonia Congenita (MC, also Thomsen and Becker Disease), Paramyotonia Congenita (PC), Central Core Disease (CCD), Nemaline Myopathy (NM)

Myotubular Myopathy/Centronuclear Myopathy (MTM or CNM), Periodic Paralysis (PP, two forms: Hypokalemic and Hyperkalemic).

By "MND" is meant a disease affecting a neuron with motor function, i. e. , a neuron that conveys motor impulses. Such neurons are also termed "motor neurons". These neurons include alpha neurons of the anterior spinal cord that give rise to the alpha fibers which innervate the skeletal muscle fibers; beta neurons of the anterior spinal cord that give rise to the beta fibers which innervate the extrafusal and intrafusal muscle fibers; gamma neurons of the anterior spinal cord that give rise to the gamma (fusimotor) fibers which innervate the intrafusal fibers of the muscle spindle; heteronymous neurons that supply muscles other than those from which afferent impulses originate; homonymous neurons that supply muscles from which afferent impulses originate; lower peripheral neurons whose cell bodies lie in the ventral gray columns of the spinal cord and whose terminations are in skeletal muscles; peripheral neurons that receive impulses from interneurons; and upper neurons in the cerebral cortex that conduct impulses from the motor cortex to motor nuclei of the cerebral nerves or to the ventral gray columns of the spinal cord.

Examples of motoneuron disorders include the various amyotrophies such as hereditary amyotrophies including hereditary spinal muscular atrophy, acute infantile spinal muscular atrophy such as Werdnig-Hoffman disease, progressive muscular atrophy in children such as the proximal, distal type and bulbar types, spinal muscular atrophy of adolescent or adult onset including the proximal, scapuloperoneal, facioscapulohumeral and distal types, amyotrophic lateral sclerosis (ALS) and primary lateral sclerosis (PLS). Also included within the term is motoneuron injury.

The term "Amyotrophic Lateral Sclerosis" (or "ALS"), also called Lou Gehrig's disease, is a fatal disease affecting motor neurons of the cortex, brain stem and spinal cord. (Hirano, (1996) Neurology, 47(4 Suppl. 2): S63-6). Although the etiology of the disease is unknown, one theory is that neuronal cell death in ALS is the result of over-excitement of neuronal cells due to excess extracellular glutamate. Glutamate is a neurotransmitter that is released by glutaminergic neurons, and is taken up into glial cells where it is converted into glutamine by the enzyme glutamine synthetase, glutamine then re-enters the neurons and is hydrolyzed by glutaminase to form glutamate, thus replenishing the neurotransmitter pool. In a normal spinal cord and brain stem, the level of extracellular glutamate is kept at low micromolar levels in the extracellular fluid because glial cells, which function in part to support neurons, use the excitatory amino acid transporter type 2 (EAAT2) protein to absorb glutamate immediately. A deficiency in the normal EAAT2 protein in patients with ALS, was identified as being important in the pathology of the disease {See e.g., Meyer et al. (1998) J. Neurol. Neurosurg. Psychiatry, 65: 594-596; Aoki et al. (1998) Ann. Neurol. 43: 645-653; Bristol et al. (1996) Ann Neurol. 39: 676-679). One explanation for the reduced levels of EAAT2 is that EAAT2 is spliced aberrantly (Lin et al. (1998) Neuron, 20: 589-602). The aberrant splicing produces a splice variant with a deletion of 45 to 107 amino acids located in the C-terminal region of the EAAT2 protein (Meyer et al. (1998) Neurosci Lett. 241: 68-70). Due to the lack of, or defectiveness of EAAT2, extracellular glutamate accumulates, causing neurons to fire continuously. The accumulation of glutamate has a toxic effect on neuronal cells because continual firing of the neurons leads to early cell death. Although a great deal is known about the pathology of ALS little is known about the pathogenesis of the sporadic form and about the causative properties of mutant SOD protein in familial ALS (Bruijn, et al. (1996) Neuropathol. Appl. Neurobiol, 22: 373-87; Bruijn, et al. (1998) Science 281: 1851-54). Many models have been speculated, including glutamate toxicity, hypoxia, oxidative stress, protein aggregates, neurofilament and mitochondrial dysfunction Cleveland, et al. (1995) Nature 378: 342-43; Cleveland, et al. Neurology, 47(4 Suppl. 2): S54-61, discussion S61-20996); Cleveland, (1999) Neuron, 24: 515-20; Cleveland, et al. (2001) Nat. Rev. Neurosci., 2: 806-19; Couillard-Despres, et al. (1998) Proc. Natl. Acad. ScL USA, 95: 9626-30; Mitsumoto, (1997) Ann. Pharmacother., 31 : 779-81; Skene, et al. (2001) Nat. Genet. 28: 107-8; Williamson, et al (2000) Science, 288: 399).
Presently, there is no cure for ALS, nor is there a therapy that has been proven effective to prevent or reverse the course of the disease. Several drugs have recently been approved by the Food and Drug Administration (FDA). To date, attempts to treat ALS have involved treating neuronal degeneration with long-chain fatty alcohols which have cytoprotective effects (See U.S. Pat. No. 5,135,956); or with a salt ofpyruvic acid (See U.S. Pat. No. 5,395,822); and using a glutamine synthetase to block the glutamate cascade (See U.S. patent 5,906,976). For example, Riluzole, a glutamate release inhibitor, has been approved in the U.S. for the treatment of ALS, and appears to extend the life of at least some patients with ALS by three months. However, some reports have indicated that even though Riluzole therapy marginally prolongs survival time, it does not appear to provide any improvement of muscular strength in the patients. Therefore, the effect of Riluzole is limited in that the therapy does not modify the quality of life for the patient (Borras-Blasco etal. (1998) Rev. Neurol, 27: 1021-1027).

As used hereinafter, "molecular weight" means the mean molecular weight of the PEG.

"PEG or PEG group" according to the invention means a residue containing poly( ethylene glycol) as an essential part. Such a PEG can contain further chemical groups which are necessary for binding reactions; which results from the chemical synthesis of the molecule; or which is a spacer for optimal distance of the parts of the molecule from one another. In addition, such a PEG can consist of one or more PEG side-chains which are linked together. PEG groups with more than one PEG chain are called multiarmed or branched PEGs. Branched PEGs can be prepared, for example, by the addition of polyethylene oxide to various polyols, including glycerol, pentaerythriol, and sorbitol. For example, a four-armed branched PEG can be prepared from pentaerythriol and ethylene oxide. Branched PEGs usually have 2 to 8 arms and are described in, for example, EP-A 0 473 084 and US Patent No. 5,932,462. Especially preferred are PEGs with two PEG side-chains (PEG2) linked via the primary amino groups of a lysine (Monfardini, C, et al., Bioconjugate Chem. 6 (1995) 62-69).

"Substantially homogeneous" as used herein means that the only PEGylated IGF-I variant molecules produced, contained or used are those having one or two PEG group(s) attached. The preparation may contain small amounts of unreacted (i.e., lacking PEG group) protein. As ascertained by peptide mapping and N-terminal sequencing, one example below provides for the preparation which is at least 90% PEG-IGF-I variant conjugate and at most 5% unreacted protein. Isolation and purification of such homogeneous preparations of PEGylated IGF-I variant can be performed by usual purification methods, preferably size exclusion chromatography.

"MonoPEGylated" as used herein means that IGF-I variant is PEGylated at only one lysine per IGF-I variant molecule, whereby only one PEG group is attached covalently at this site. The pure monoPEGylated IGF-I variant (without N-terminal PEGylation) is at least 80% of the preparation, preferably 90%, and most preferably, monoPEGylated IGF-I variant is 92%, or more, of the preparation, the remainder being e.g. unreacted (non-PEGylated) IGF-I and/or N-terminally PEGylated IGF-I variant. The monoPEGylated IGF-I variant preparations according to the invention are therefore homogeneous enough to display the advantages of a homogeneous preparation, e.g., in a pharmaceutical application. The same applies to the diPEGylated species.

"PEGylated IGF-I variant" or "amino-reactive PEGylation" as used herein means that a IGF-I variant is covalently bound to one or two poly( ethylene glycol) groups by amino-reactive coupling to one or two lysines of the IGF-I variant molecule. The PEG group(s) is/are attached at the sites of the IGF-I variant molecule that are the primary [epsilon]-amino groups of the lysine side chains. It is further possible that PEGylation occurs in addition on the N-terminal [alpha]-amino group. Due to the synthesis method and variant used, PEGylated IGF-I variant can consist of a mixture of IGF-I variants, PEGylated at K65, K68 and/or K27 with or without N- terminal PEGylation, whereby the sites of PEGylation can be different in different molecules or can be substantially homogeneous in regard to the amount of poly(ethylene glycol) side chains per molecule and/or the site of PEGylation in the molecule. Preferably the IGF-I variants are mono- and/or diPEGylated and especially purified from N-terminal PEGylated IGF-I variants.

"PEG or poly(ethylene glycol)" as used herein means a water soluble polymer that is commercially available or can be prepared by ring-opening polymerization of ethylene glycol according to methods well known in the art (Kodera, Y., et al., Progress in Polymer Science 23 (1998) 1233-1271; Francis, G.E., et al., Int. J. Hematol. 68 (1998) 1-18. The term "PEG" is used broadly to encompass any polyethylene glycol molecule, wherein the number of ethylene glycol (EG) units is at least 460, preferably 460 to 2300 and especially preferably 460 to 1840 (230 EG units refers to an molecular weight of about 10 kDa). The upper number of EG units is only limited by solubility of the PEGylated IGF-I variants. Usually PEGs which are larger than PEGs containing 2300 units are not used. Preferably, a PEG used in the invention terminates on one end with hydroxy or methoxy (methoxy PEG, mPEG) and is on the other end covalently attached to a linker moiety via an ether oxygen bond. The polymer is either linear or branched. Branched PEGs are e.g. described in Veronese, F.M., et al., Journal ofBioactive and Compatible Polymers 12 (1997) 196-207.

An aspect of the present invention relates to a PEGylated IGF - I variant according to the claims for use in a method for the treatment of neuromuscular disorders, preferably a motor neuron disease and most preferably ALS by administering a pharmaceutically effective amount of said PEGylated IGF-I variant to a patient in need thereof In an even more preferred embodiment, the disease to be treated is ALS which is caused by a genetic defect that leads to mutation of the superoxide dismutase 1.

This PEGylated IGF-I variant is characterized in that PEG is attached to a lysine residue of a recombinant human IGF-I mutein which carries one or two amino acid alterations at amino acid positions 27, 65 and 68 of the wild-type human IGF-I amino acid sequence (SEQ ID NO: 1) so that one or two of amino acids at positions 27, 65 and 68 is/are a polar amino acid but not lysine.

A "polar amino acid" as used herein refers to an amino acid selected from the group consisting of cysteine (C), aspartic acid (D), glutamic acid (E), histidine (H), asparagine (N), glutamine (Q), arginine (R), serine (S), and threonine (T). Lysine is also a polar amino acid, but excluded, as lysine is replaced according to the invention. Arginine is preferably used as polar amono acid.

Preferred are PEGylated forms of recombinant human IGF-I muteins that are characterized by the following amino acid alterations of the wild-type IGF-I amino acid sequence (SEQ ID NO: 1):
(a) K65R and K68R (SEQ ID NO: 2)
(b) K27R and K68R (SEQ ID NO: 3)
(c) K27R and K65R (SEQ ID NO: 4).

Special preference is given to the PEGylated form of the recombinant human IGF-I mutein with amino acid alterations K27R and K65R (SEQ ID NO: 4) characterized by mono-PEGylation at K68.

Preference is also given to compositions of a lysine-PEGylated IGF-I variant as described above and a IGF-I variant which is N-terminally PEGylated, wherein said IGF-I variants are identical in terms of the primary amino acid sequence and in that they carry one or two amino acid alterations at amino acid positions 27, 65 and 68 of the wild-type human IGF-I amino acid sequence (SEQ ID NO: 1) so that one or two of amino acids at positions 27, 65 and 68 is/are a polar amino acid but not lysine. Preferably the molecular ratio is 9 : 1 to 1 : 9 (ratio means lysine-PEGylated IGF-I variant / N-terminally PEGylated IGF-I variant). Further preferred is a composition wherein the molar ratio is at least 1 : 1 (at least one part lysine-PEGylated IGF-I variant per one part of N-terminally PEGylated IGF-I variant), preferably at least 6 : 4 ( at least six parts lysine-PEGylated IGF-I variant per four parts of N-terminally PEGylated IGF-I variant). Preferably both the lysine- PEGylated IGF-I variant and the N-terminally PEGylated IGF-I variant are monoPEGylated. Preferably in this composition the variant is identical in both the lysine-PEGylated IGF-I variant and the N-terminally PEGylated IGF-I variant. The IGF-I variant is preferably selected from IGF-I muteins having the following amino acid alterations of the wild-type human IGF-I amino acid sequence (SEQ ID NO: 1):
(a) K65R and K68R (SEQ ID NO: 2)
(b) K27R and K68R (SEQ ID NO: 3)
(c) K27R and K65R (SEQ ID NO: 4).

Preferred PEGylated forms of recombinant human IGF-I muteins according to SEQ ID NOS 2 to 4 are obtainable when following the procedure for producing of a lysine-PEGylated IGF-I or a lysine-PEGylated IGF-I variant, said variant comprising one or two amino acid(s) selected from the group consisting of lysine 27, 65 and/or 68 substituted independently by another polar amino acid as described in WO 2008/025528 A1 incorporated . The process(es) described in WO 2008/025528 A1 allow(s) the preparation of recombinant human IGF-I muteins according to SEQ ID Nos 2 to 4, which do not bear N-terminal PEGylation.

It is further preferred, that the PEGylated IGF-I variant is a variant in which up to three (preferably all three) amino acids at the N-terminus are truncated. The respective wild type mutant is named Des( 1-3)-IGF-I and lacks the amino acid residues glycine, proline and glutamate from the N-terminus (Kummer, A., et al., Int. J. Exp. Diabesity Res. 4 (2003) 45-57).

Preferably the poly(ethylene glycol) group(s) have an overall molecular weight of at least 20 kDa, more preferably from about 20 to 100 kDa and especially preferably from 20 to 80 kDa. The poly( ethylene glycol) group(s) is/are either linear or branched.

Amino-reactive PEGylation as used herein designates a method of randomly attaching poly( ethylene glycol) chains to primary lysine amino group(s) of the IGF- I variant by the use of reactive (activated) poly(ethylene glycol), preferably by the use ofN-hydroxysuccinimidyl esters of, preferably, methoxypoly( ethylene glycol). The coupling reaction attaches poly(ethylene glycol) to reactive primary [epsilon]-amino groups of lysine residues and optionally the [alpha]-amino group of the N-terminal amino acid of IGF-I. Such amino group conjugation of PEG to proteins is well known in the art. For example, review of such methods is given by Veronese, F.M., Biomaterials 22 (2001) 405-417. According to Veronese, the conjugation of PEG to primary amino groups of proteins can be performed by using activated PEGs which perform an alkylation of said primary amino groups. For such a reaction, activated alkylating PEGs, for example PEG aldehyde, PEG-tresyl chloride or PEG epoxide can be used. Further useful reagents are acylating PEGs such as hydroxysuccinimidyl esters of carboxylated PEGs or PEGs in which the terminal hydroxy group is activated by chloroformates or carbonylimidazole. Further useful PEG reagents are PEGs with amino acid arms. Such reagents can contain the so-called branched PEGs, whereby at least two identical or different PEG molecules are linked together by a peptidic spacer (preferably lysine) and, for example, bound to IGF-I variant as activated carboxylate of the lysine spacer. Mono-N-terminal coupling is also described by Kinstler, O., et al., Adv. Drug Deliv. Rev. 54 (2002) 477-485.

Useful PEG reagents are e.g. available from Nektar Therapeutics Inc.

Any molecular mass for a PEG can be used as practically desired, e.g., from about 20 kDa to 100 kDa (n is 460 to 2300). The number of repeating units "n" in the PEG is approximated for the molecular mass described in Daltons. For example, if two PEG molecules are attached to a linker, where each PEG molecule has the same molecular mass of 10 kDa (each n is about 230), then the total molecular mass of PEG on the linker is about 20 kDa. The molecular masses of the PEG attached to the linker can also be different, e.g., of two molecules on a linker one PEG molecule can be 5 kDa and one PEG molecule can be 15 kDa. Molecular mass means always average molecular mass.

Suitable processes and preferred reagents for the production of amino- reactive PEGylated IGF-I variants are described in WO 2006/066891. It is understood that modifications, for example, based on the methods described by Veronese, F.M., Biomaterials 22 (2001) 405-417 , can be made in the procedures as long as the process results in PEGylated IGF-I variants described above. Particularly preferred processes for the preparation of PEGylated IGF-I variants according to present invention are described in WO 2008/025528 A1 .

The occurrence of up to three potentially reactive primary amino groups in the target protein (up to two lysines and one terminal amino acid) leads to a series of PEGylated IGF-I variants isomers that differ in the point of attachment of the poly(ethylene glycol) chain.

PEGylated IGF-I variants contain one or two PEG groups linear or branched and randomly attached thereto, whereby the overall molecular weight of all PEG groups in the PEGylated IGF-I variant is preferably about 20 to 80 kDa. Small deviations from this range of molecular weight are possible. However, it is expected that activity decreases as the molecular weight increases due to reduced IGF-I receptor activation and blood-brain barrier transport. Therefore, the range of 20 to 100 kDa for the molecular weight of PEG has to be understood as the optimized range for a conjugate of PEG and IGF-I variant useful for an efficient treatment of MND, in particular ALS.

### Pharmaceutical Formulations

The PEGylated IGF-I variants described hereinbefore are characterized by an improved stability in the circulation enabling a sustained access to IGF-I receptors throughout the body with low application intervals, i.e. prolonged intervals.

PEGylated IGF-I variants can be formulated according to methods for the preparation of pharmaceutical compositions which methods are known to the person skilled in the art. For the production of such compositions, a PEGylated IGF-I variant according to the invention is combined in a mixture with a pharmaceutically acceptable carrier, preferably by dialysis against an aqueous solution containing the desired ingredients of the pharmaceutical compositions.
Such acceptable carriers are described, for example, in Remington's Pharmaceutical Sciences, 18th edition, 1990, Mack Publishing Company, edited by Oslo et al. (e.g. pp. 1435-1712). Typical compositions contain an effective amount of the substance according to the invention, for example from about 0.1 to 100 mg/ml, together with a suitable amount of a carrier. The compositions may be administered parenterally. The PEGylated IGF-I according to the invention is administered preferably via intraperitoneal, subcutaneous, intravenous or intranasal application.

The pharmaceutical formulations according to the invention can be prepared according to known methods in the art. Usually, solutions of PEGylated IGF-I variant are dialyzed against the buffer intended to be used in the pharmaceutical composition and the desired final protein concentration is adjusted by concentration or dilution.

Such pharmaceutical compositions may be used for administration for injection or infusion, preferably via intraperitoneal, subcutaneous, intravenous or intranasal application and contain an effective amount of the PEGylated IGF-I variant together with pharmaceutically acceptable diluents, preservatives, solubilizers, emulsifiers, adjuvants and/or carriers. Such compositions include diluents of various buffer contents (e.g. arginine, acetate, phosphate), pH and ionic strength, additives such as detergents and solubilizing agents (e.g. Tween(TM) 80/polysorbate, pluronic(TM) F68), antioxidants (e.g. ascorbic acid, sodium metabisulfite), preservatives (Timersol(TM), benzyl alcohol) and bulking substances (e.g. saccharose, mannitol), incorporation of the material into particulate preparations of polymeric compounds such as polylactic acid, polyglycolic acid, etc. or into liposomes. Such compositions may influence the physical state stability rate of release and clearance of PEGylated IGF-I variants.

### Dosages and Drug Concentrations

Typically, in a standard treatment regimen, patients are treated with dosages in the range between 0.001 to 20 mg, preferably 0.01 to 8 mg of PEGylated IGF-Ivariant per kg per week over a certain period of time, lasting from one week to about 3 months or even longer. Drug is applied as a single weekly s.c., i.v. or i.p. (intraperitoneal) bolus injection or infusion of a pharmaceutical formulation containing 0.1 to 100 mg of a PEGylated IGF-I variant described hereinbefore per ml. This treatment can be combined with any standard (e.g. chemotherapeutic) treatment, by applying PEGylated IGF-I before, during or after the standard treatment. This results in an improved outcome compared to standard treatment alone.

It was found, that the PEGylated IGF-I variants described hereinbefore can be administered only one or two times per week for successful treatment. A method for the treatment of a neuromuscular disorder, preferably an MND, and even more preferred ALS should therefore comprise administering to a patient in need thereof a therapeutically effective amount of a PEGylated IGF-I variant described hereinbefore with one dosage each in the range between 0.001 to 3 mg, preferably 0.01 to 3 mg of PEGylated IGF-I variant per kg and per 3-8 days, preferably per 7 days. As PEGylated IGF-I variant is preferably a monoPEGylated IGF-I variant used.

The PEGylated IGF-I variants described hereinbefore can be used for the preparation of a medicament for the treatment of a neuromuscular disorder, preferably an MND, and even more preferred ALS which is administered to a patient in need thereof in a therapeutically effective amount and with one or two dosages in the range between 0.001 to 3 mg, preferably 0.01 to 3 mg of PEGylated IGF-I variant per kg and per 6-8 days, preferably per 7 days. As PEGylated IGF-I variant is preferably a monoPEGylated IGF-I variant used.

The PEGylated IGF-I variants described hereinbefore can also be used separately, sequentially or simultaneously in a combination combined with a second pharmacologically active compound for the treatment of a neuromuscular disorder, preferably an MND, and even more preferred ALS. Preferably, the second pharmacologically active compound of the combination is at least one neuroprotectant having an inhibitory effect on glutamate release or the effect of inactivation of voltage-dependent sodium channels or the ability to interfere with intracellular events that follow transmitter binding at excitatory amino acid receptors.

The second pharmacologically active compound is preferably riluzole. Riluzole blocks TTX-S sodium channels, which are associated with damaged neurons (Song JH, Huang CS, Nagata K, Yeh JZ, Narahashi T. Differential action of riluzole on tetrodotoxin-sensitive and tetrodotoxin-resistant sodium channels J. Pharmacol. Exp. Ther. 1997; 282: 707-14). This reduces influx of calcium ions and indirectly prevents stimulation of glutamate receptors. Together with direct glutamate receptor blockade, the effect of the neurotransmitter glutamate on motor neurons is reduced.

The term "riluzole" as used herein refers to 2-amino-6-(trifluoromethoxy)benzothiazole, 6-(trifluoromethoxy)benzothiazol-2-amine or CAS-1744-22-5. In a broader sense of this embodiment, the term "riluzole" also comprises active ingredients having at least one pharmacological property also observed with riluzole selected from an inhibitory effect on glutamate release, inactivation of voltage-dependent sodium channels and the ability to interfere with intracellular events that follow transmitter binding at excitatory amino acid receptors. The use of riluzole in ALS is described in US 5,527, 814, the compound and its preparation is disclosed in EP 050 551. Other neuroprotectant compounds can be prepared as described, e. g. , by Yagupolskii et al in Zhurnal Obschei Khimii 33 (7), 2301-7 (1963).

### Sequence Listing

### SEQ ID NO: 1

Amino acid sequence of wild-type human IGF-I (amino acids 1-70 of IGF-I precursor protein according to SwissProt P01343).

### SEQ ID NO: 2

Amino acid sequence of human IGF-I mutein carrying amino acid exchanges K65R and K68R.

### SEQ ID NO: 3

Amino acid sequence of human IGF-I mutein carrying amino acid exchanges K27R and K68R.

### SEO ID NO: 4

Amino acid sequence of human IGF-I mutein carrying amino acid exchanges K27R and K65R.

### Brief Description of the Figures

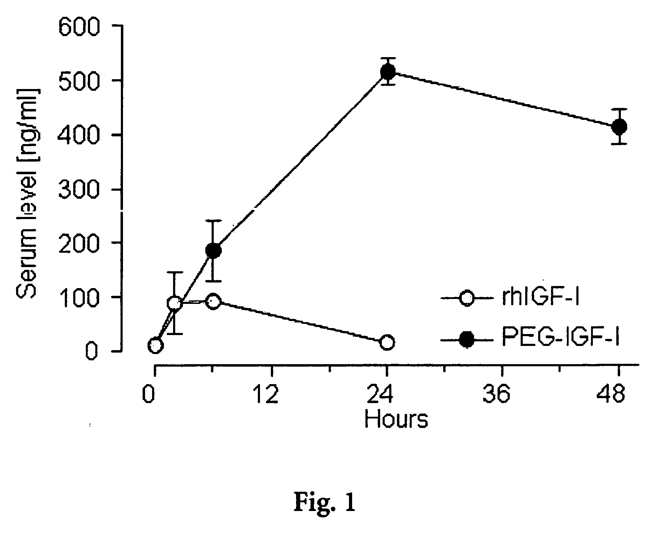

Fig. 1 shows serum detection after s.c. injection of 100 µg/kg rhIGF-I or PEG-IGF-I in mice. Serum levels of PEG-IGF-I or rhIGF-I were detected at indicated time points by ELISA techniques.

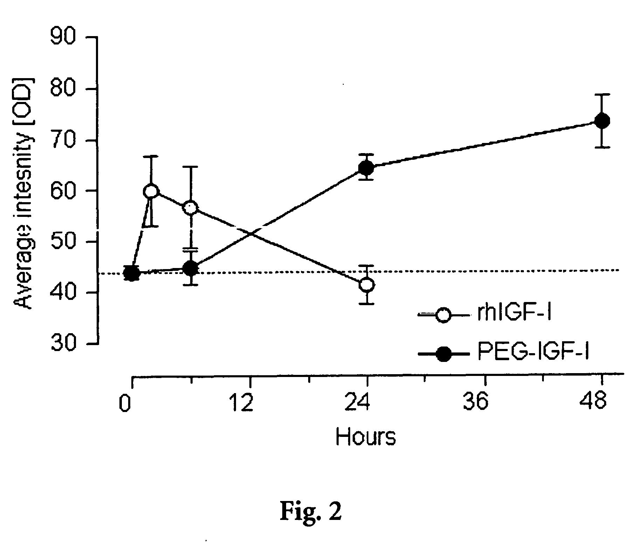

Fig. 2 shows IGF-I immunoreactivity in CA1 neurons of the hippocampus after s.c. injection ofrhIGF-I or PEG-IGF-1 (100 µg/kg) in mice. At indicated time points, brains were removed and immunostained for hIGF-I. Digital images from the CA1 region of the hippocampus were analyzed for staining intensity within neurons.

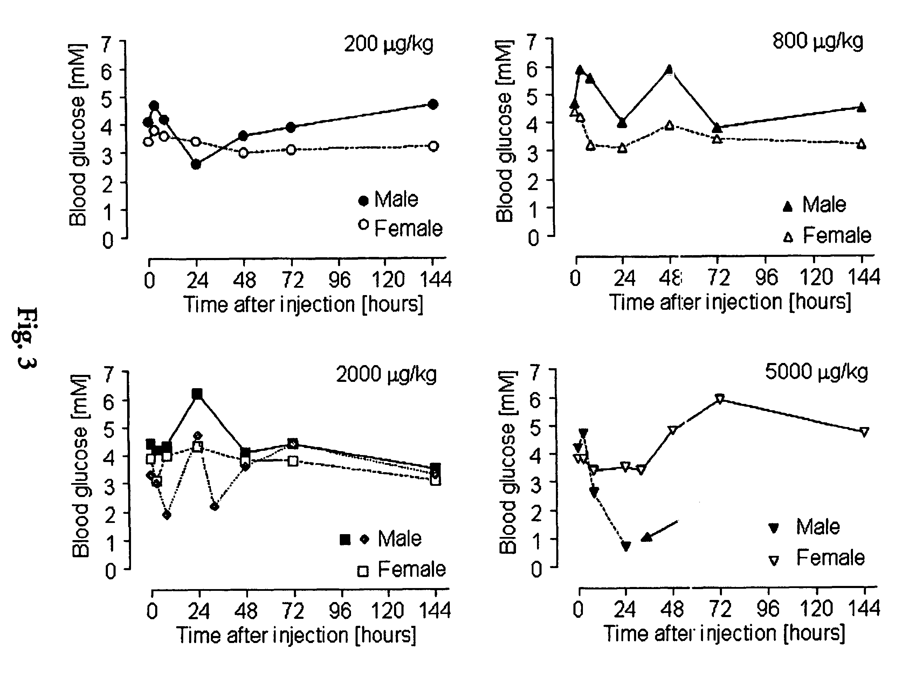

Fig. 3 shows plasma glucose levels after s.c. injection of PEG-IGF-I (200-5000 µg/kg) in beagle dogs. Glucose levels were estimated from blood drops at the respective time points using the Roche AkkuCheck device. The arrow indicates the only significant occurrence of severe hypoglycemia in the male dog at the 5000 µg/kg dose.

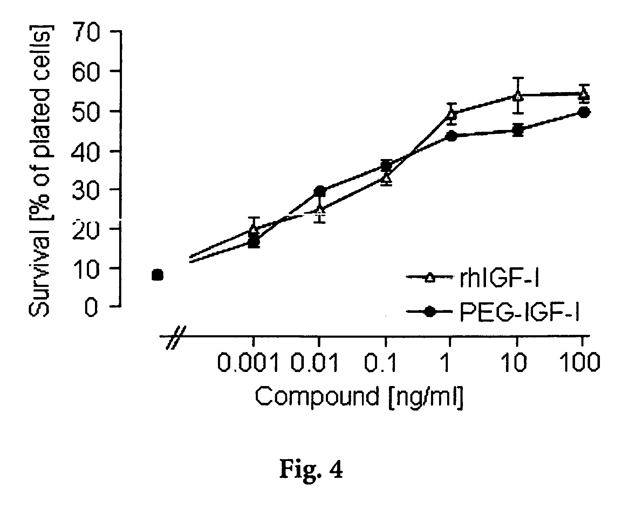

Fig. 4 shows *in vitro* survival of mouse primary motoneurons after 5 days treatment with rhIGF-I or PEG-IGF-I. Primary motoneurons from C57B1/6 mice were cultivated in presence or absence of PEG-IGF-I or rhIGF-I at different concentrations and survival estimated by phase contrast microscopy at 5 days in vitro.

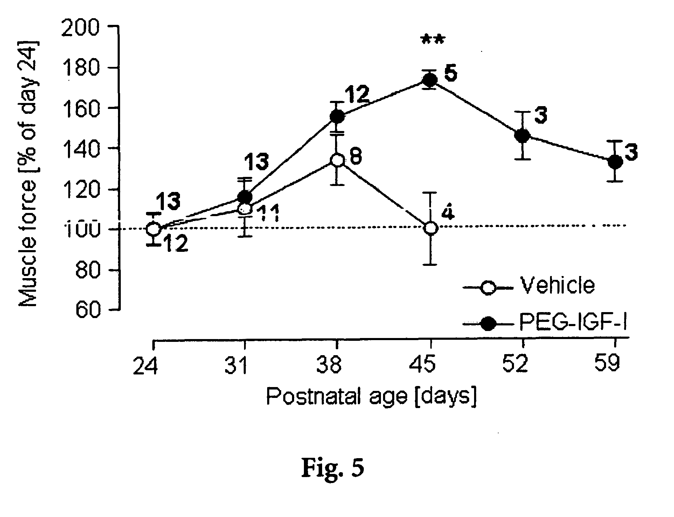

Fig. 5 shows grip strength ofpmn mice treated with vehicle or 150 µg/kg PEG-IGF-I s.c. q2d. Animals were tested weekly for muscle force of fore limbs, numbers indicate animals analysed per time point (**, p<0.01).

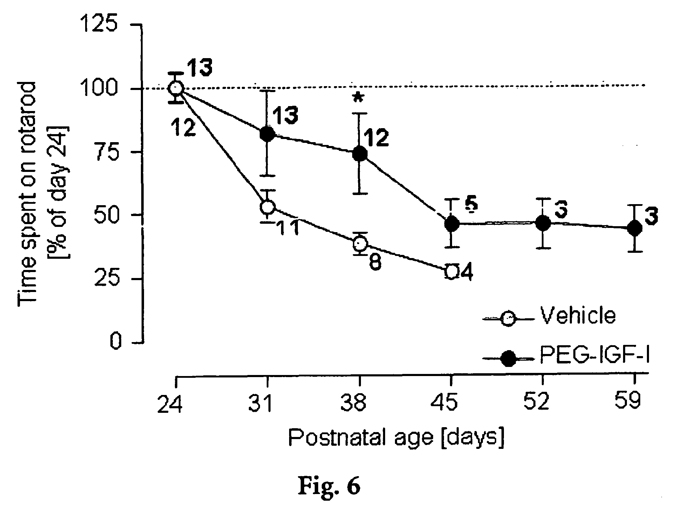

Fig. 6 shows rotarod performance of pmn mice treated with vehicle or 150 µg/kg PEG-IGF-I s.c. q2d. Animals were tested weekly for motor coordination, numbers indicate animals per time point (*, p<0.05).

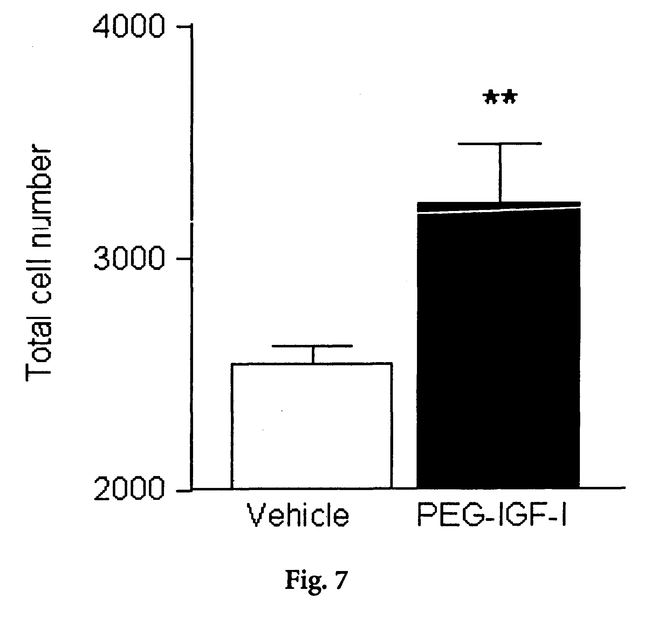

Fig. 7 shows motoneuron survival in the facial nucleus of pmn mice treated with vehicle or PEG-IGF-1 (150 µg/kg s.c. q2d). Animals were killed at postnatal day 34 and tissue processed for histology. Stereological examination of motoneuron numbers was performed blinded, values express total numbers per mouse (**, p<0.01).

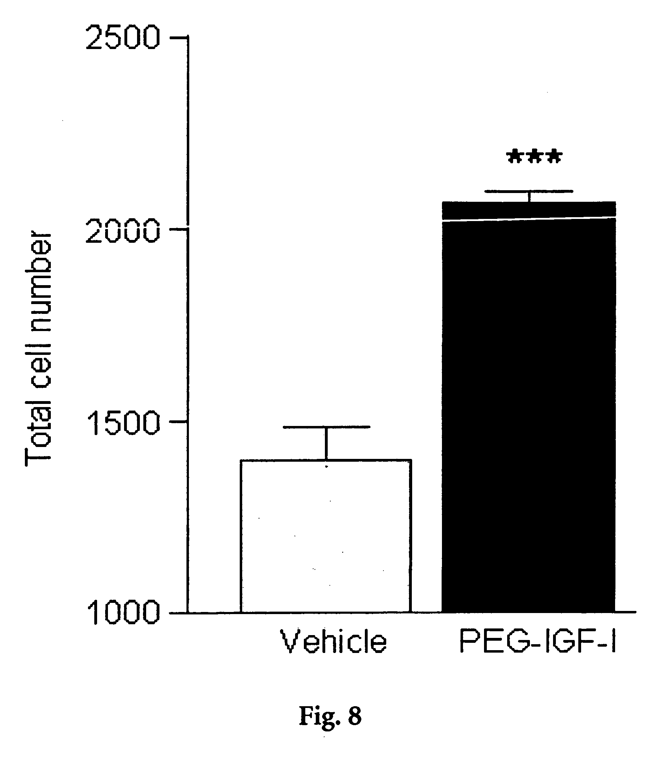

Fig. 8 shows motoneuron survival in the lumbar spinal cord of pmn mice treated with vehicle or PEG-IGF-I (150 µg/kg s.c. q2d). Animals were killed at postnatal day 34 and tissue processed for histology. Stereological examination of motoneuron numbers was performed blinded, values express total numbers per mouse (***, p<0.001).

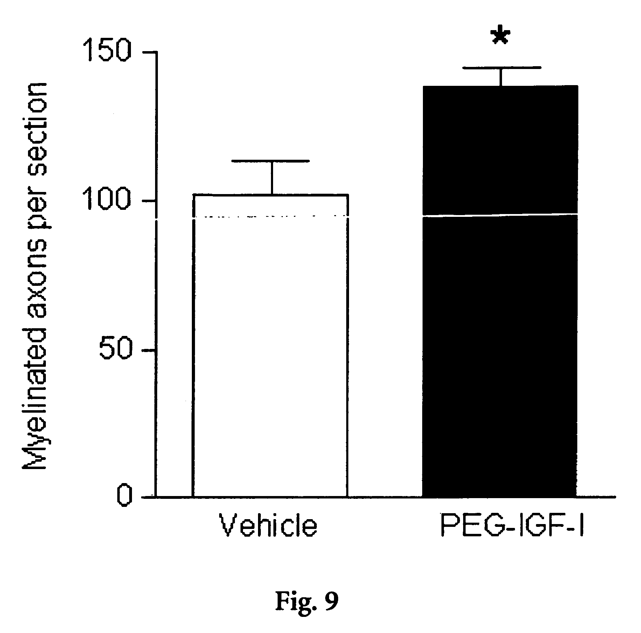

Fig. 9 shows myelinated axon numbers in the proximal phrenic nerve of pmn mice treated with vehicle or PEG-IGF-I (150 µg/kg s.c. q2d). Animals were killed at postnatal day 34 and tissue processed for histology. Stereological examination of numbers of myelinated axons was performed blinded, values express total numbers per phrenic nerve (*, p<0.05).

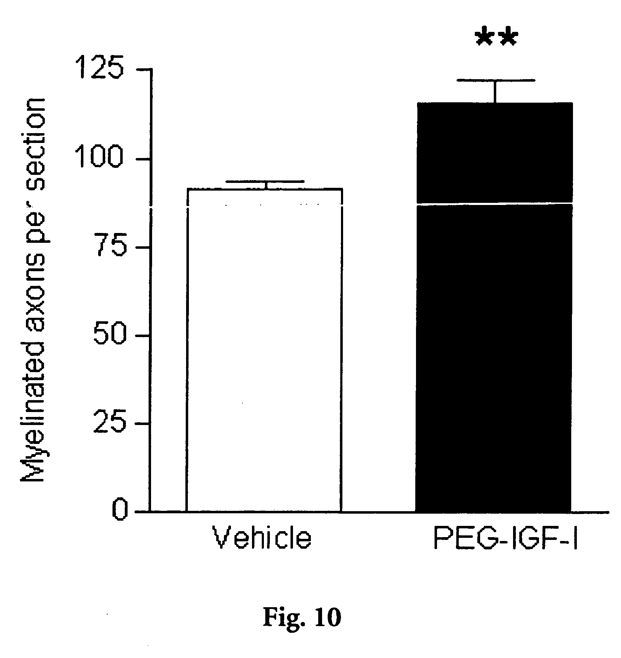

Fig. 10 shows myelinated axon numbers in the distal phrenic nerve ofpmn mice treated with vehicle or PEG-IGF-I (150 µg/kg s.c. q2d). Animals were killed at postnatal day 34 and tissue processed for histology. Stereological examination of numbers of myelinated axons was performed blinded, values express total numbers per phrenic nerve (**, p<0.01).

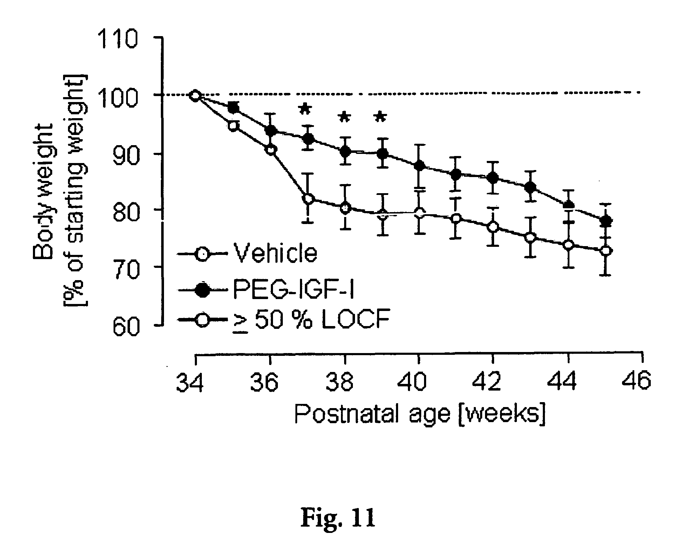

Fig. 11 shows body weight analysis of SOD1(G93A) mice treated with vehicle or PEG-IGF-1 (150 µg/kg s.c. q3.5d). Body weight was assessed weekly and values were normalized for the body weight at first examination which was set to 100 % (*, p<0.05).

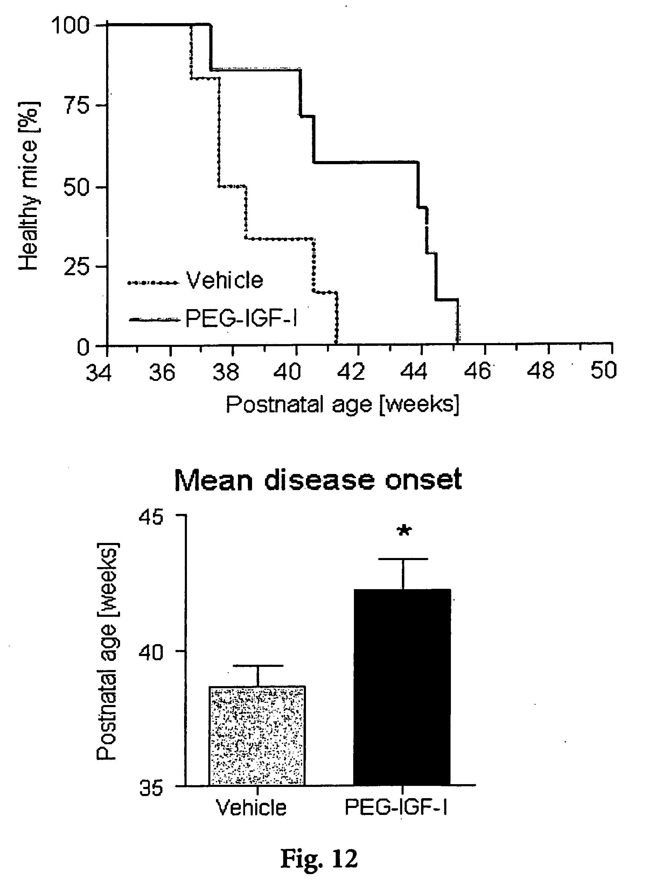

Fig. 12 shows disease onset in SOD1(G93A) mice treated with vehicle or PEG-IGF-1 (150 µg/kg s.c. q3.5d). Animals were examined weekly and disease onset defined by hindlimb weakness, abnormal gait and difficulty to hold onto an inverted wire mesh. The Kaplan-Meier plot shows disease onset in individual mice treated from postnatal week 34 on. The bar graph shows the average age at disease onset for both groups (p<0.05).

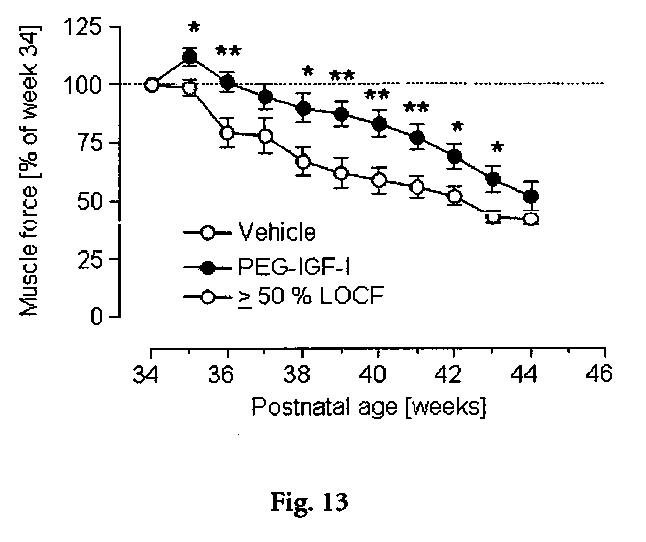

Fig. 13 shows grip strength of SOD1(G93A) mice treated with vehicle or PEG-IGF-1 (150 µg/kg s.c. q3.5d). Animals were tested weekly for muscle force of fore limbs. LOCF analysis of animals dying during the time course was performed by including the last measured values into the further data (*, p<0.05; **, p<0.01).

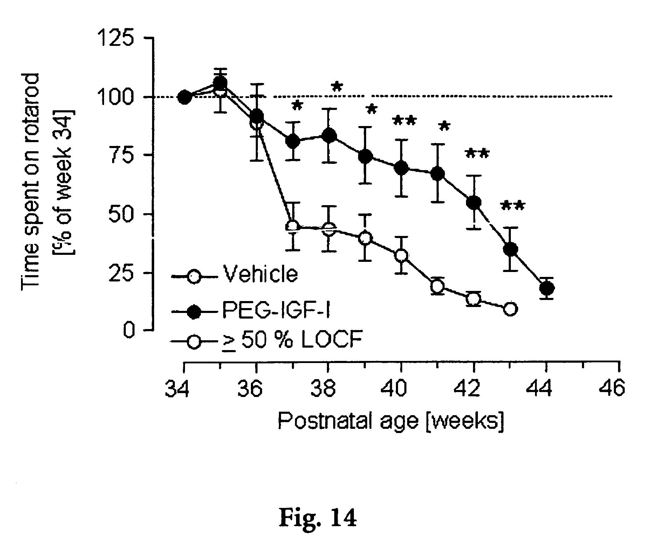

Fig. 14 shows rotarod performance of SOD1(G93A) mice treated with vehicle or PEG-IGF-I (150 µg/kg s.c. q3.5d). Animals were tested weekly for motor coordination. LOCF analysis of animals dying during the time course was performed by including the last measured values into the further data (*, p<0.05; **, p<0.01).

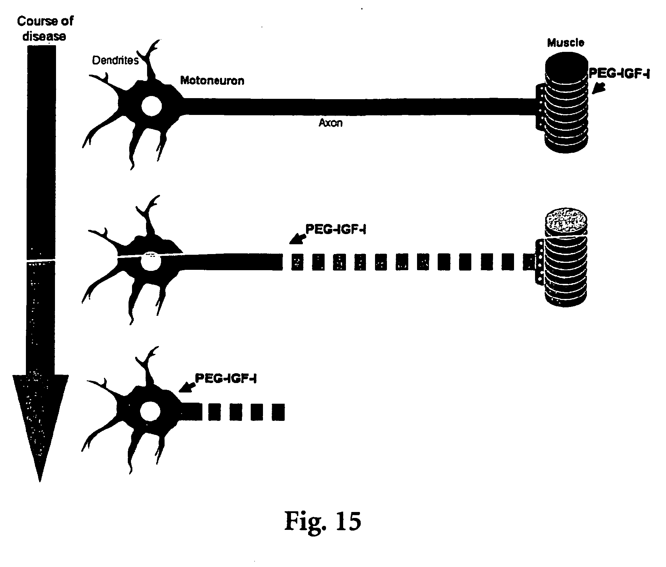

Fig. 15 shows *in vivo* actions of PEG-IGF-I related to the neuromuscular unit as demonstrated in the ALS mouse models. PEG-IGF-I was shown to improve the neuromuscular function as well protect motor axons and motoneurons in the brain stem and spinal cord and therefore is suggested to act on all parts responsible for maintaining the neuromuscular junction.

### Methods:

### Mouse embryonic motoneuron cultures

Cultures of spinal motoneurons from embryonic day 12.5 mice were prepared by a panning technique using a monoclonal rat anti-p75 antibody (Chemicon, Hofheim, Germany). The ventrolateral parts of individual lumbar spinal cords were dissected and transferred to HBSS containing 10 µM 2-mercaptoethanol. After treatment with trypsin (0.05%, 10 min), single-cell suspensions were generated by trituration. The cells were plated on a rat anti-p75 coated culture dish (Greiner, Nürtingen, Germany) and left at room temperature for 30 min. The individual wells were subsequently washed with Hank's balanced salt solution (HBSS; 3 times), and the attaching cells were then isolated from the plate with depolarizing saline (0.8% NaCl, 35 mM KCl and 1 µM 2-mercaptoethanol). Cells were plated at a density of 3000 cells/well in 4-well culture dishes (Greiner), precoated with poly-ornithine and laminin as described (Miller, T. M. et al., J. Biol. Chem. 272, 9847-9853, 1997). Cells were grown in Neurobasal medium (Life Technologies, Karlsruhe, Germany), B27 supplement, 10% horse serum, 500 µM glutamax and 50 µg/ml apotransferrin at 37°C in a 5% CO₂ atmosphere. Fifty percent of the medium was first replaced at day one and then every second day. Initial counting of plated cells was done when all cells were attached to the culture dish, after 4 hours. Phase bright cells were then additionally counted at day five. Ten fields (1.16 mm²/field) were counted in each well at each time point.

### Serum rhIGF-I or PEG-IGF-I levels and intraneuronal IGF-I staining in CA1 neurons

For estimation of serum rhIGF-I or PEG-IGF-I levels, blood samples from C57B1/6 mice were taken at different time points (n=4 mice per time point) after a single s.c. injection of either 100 µg/kg rhIGF-I or PEG-IGF-I. Serum was prepared and processed by ELISA assays. For the detection of rhIGF-I, a commercial rhIGF-I assay (DSL) was used. For detection of PEG-IGF-I, streptavidine-coated assay microplates were coated with a biotinylated anti-PEG (IgM) capture antibody. Serum samples were incubated for 15 h with digoxygenated IGFBP-4 to replace any IGF-I bound by endogenous IGFBP's by IGFBP-4. After washing, the plates were incubated with anti-Dig-POD (Fab) and detected by ABTS colour reaction. Absorbance signals were quantified with the SpectraMax M2^{e} reader at 405 nm and 490 nm.

At different time points after a single s.c. injection of either 100 µg/kg rhIGF-I or PEG-IGF-1, C57B1/6 mice were decapitated under isoflurane anesthesia and brains removed. Hemispheres were snap-frozen in dry ice and postfixed in paraformaldehyde (4 % in phosphate-buffered saline, PBS). Subsequently, 40 µm sagittal slices were cut with a vibratome (Zeiss). For semi-quantitative analysis of immunoreactivity, 24 slices were cut starting at 2 mm from the lateral edge. Every fourth slice was used for counting, revealing 6 slices in total per mouse. Slices were imunostained with a Goat-anti-hIGF-I antibody (R&D Systems) and counterstained with nuclear dye. Secondary detection was performed by labeling with Donkey-anti-Goat-Cy3 (Jackson). Digital pictures of CA1 neurons were assessed fully blinded using a PixelFly camera (Klughammer) at identical intensity and staining intensity across the CA1 cellular layer semiautomatically aquired using the ImagePro 4.5 software (Media Cybernetics). Intensity values from 6 slices per mouse were averaged.

### Estimation of blood glucose

Beagle dogs were treated with PEG-IGF-1 (200-5000 µg/kg s.c.) and blood samples taken after different time intervals up to 6 days (144 h). Blood glucose was assessed from blood drops using the AkkuCheck device (Roche).

### Functional assessment

Mice were regularly monitored to assess disease onset which was defined when mice displayed hindlimb weakness, abnormal gait and difficulty to hold onto an inverted wire mesh. The onset of disease in SOD1(G93A) transgenic mice is variable (Gurney et al., Science 264 (5166):1772-1775, 1994) while it occurs in pmn mice during the third week after birth (Schmalbruch et al., J Neuropathol Exp Neurol 50(3):192-204, 1991). In order to assess the weakness that develops, mutant mice were subjected weekly to functional motor tests starting on postnatal day 24 (pmn mutant mice) or postnatal week 34 (SOD G93A mutant mice). The forelimb grip strength (in Newton) was recorded by averaging 5 trials on an electronic grip strength meter (Columbus Instruments, Columbus, Ohio). In addition, mice were tested for their ability to maintain balance on a rotarod apparatus (Hugo Basile Bio. Res. App.) while the rod underwent a linear acceleration from 4 to 40 rpm (rounds per minute). The time (seconds) maintained on the rod by each mouse (latency) was recorded 3 times per session. Mean values at postnatal day 24 (pmn mice) or postnatal week 34 (SOD1 mice) were considered as 100 % and results from subsequent analyses were normalized against this value.

### Histological analysis

The number of motoneuron cell bodies in the facial nucleus and lumbar spinal cord of PEG IGF-I and vehicle (i.e. respective buffer without PEG IGF-I) treated pmn mice was determined on postnatal day 34. In addition, the number of myelinated axons in the proximal and distal part of phrenic nerves was counted in these mouse mutants. Animals were transcardially perfused with 4% paraformaldehyde (PFA) in 0.1 M phosphate buffer at pH 7.4 and the brainstem and lumbar spinal cord (L1-L6) were dissected. Serial sections were cut from the brain stem region (7 µm) including the facial nuclei and from the lumbar spinal cord (12,5 µm). After Nissl staining, motoneurons were counted in every 5^{th} (facial nucleus) or 10^{th} section (spinal cord) and the raw counts were corrected for split nuclei (Masu et al., Nature 365:27-32, 1993). Phrenic nerves were postfixed overnight in 0.1 M cacodylate buffer containing 4% paraformaldehyde and 2% glutaraldehyde. After osmification and dehydration, all samples were embedded in Spurr's medium. Semithin (0,5 µm) cross sections for light microscopic examination were cut with a glass knife and stained with azur-methylenblue. The number of intact myelinated fibers was determined from photographs taken from nerve cross sections under an Leica (Nussloch, Germany) light microscope equipped with a digital camera (ActionCam; Agfa, Mortsel, Belgium).

### Examples

### Example 1:

To estimate systemic exposure of rhIGF-I and PEG-IGF-I, drug levels after single s.c. injection of 100 µg/kg rhIGF-I or PEG-IGF-I were estimated in C57B1/6 mice using specific detection assays. Thereby, PEG-IGF-I showed both a strongly prolonged half-life as well as higher serum exposure as compared to rhIGF-I (Fig. 1). To further investigate if this increased peripheral exposure translates into the brain, brain slices of these mice were immunostained with an antibody recognizing human IGF-I and intraneuronal staining in the CA1 region was assessed. IGF-I staining of CA1 neurons was increased at 2 and 6 h after s.c. injection of rhIGF-I but back to baseline levels after 24 h (Fig. 2). In contrast, increased IGF-I staining was observed at 24 and 48 h after PEG-IGF-I injection and reaching higher levels at 48 h (Fig. 2). These data show that brain entry of both rhIGFI and PEG-IGF-I shows kinetics similar to the peripheral exposure and indicates that the much higher peripheral exposure of PEG-IGF-I compared to rhIGF-I translates into better and more sustained brain entry of PEG-IGF-I compared to rhIGF-I.

### Example 2:

In toxicological tests in beagle dogs, rhIGF-I has shown a large potential to acutely induce hypoglycemia even at relatively low doses of 150 µg/kg given s.c. (NDA report 21-839). To analyse the hypoglycemic potential of PEG-IGF-I, male and female beagle dogs were treated with a single dose of PEG-IGF-I ranging from 200-5000 µg/kg s.c.. As shown in Fig. 3, up to 2000 µg/kg no consistent hypoglycemia was observed. However, at the dose of 5000 µg/kg one out of two dogs underwent a severe hypoglycemia (see arrow in Fig. 2) and had to be recovered by glucose infusion; consequently, glucose testing was stopped at this time point. Taken together, these data demonstrate that up to 2000 µg/kg s.c. PEG-IGF-I does not have a hypoglycemic potential similar to the hypoglycemia observed with rhIGF-I at 150 µg/kg (NDA report 21-839).

### Example 3:

To investigate the *in vitro* activity of PEG-IGF-I related to rhIGF-I, both compounds were compared for their efficacy on motoneuron survival. Primary motoneurons from E 12.5 aged C57B1/6 mouse embryos were cultured in the absence or presence of different concentrations of rhIGF-I or PEG-IGF-I and surviving motoneurons counted after 5 days by phase contrast microscopy. As shown in Fig. 4, both compounds showed identical efficacy on protecting motoneurons. The data indicate that rhIGF-I and PEG-IGF-I have identical biological activity.

### Example 4:

For rhIGF-I, several local or sustained dosing regimen have shown efficacy in SOD1(G93A) mice, a widely used animals model for ALS (Kaspar et al., Science 301:839, 2003; Dobrowolny et al., J Cell Biol 168:193, 2005; Nagano et al., J Neurol Sci 235:61, 2005; Narai et al., J Neurosci Res 82:452, 2005). We therefore investigated the *in vivo* efficacy of PEG-IGF-I, applied s.c. at 150 µg/kg shortly before clinical onset of disease in two independent models for ALS, pmn mice and SOD1(G93A) mice.
For testing of PEG-IGF-I in a model for sporadic ALS, pmn mice were used (Bommel et al., J Cell Biol 159:563, 2002). This ALS model develops first symptoms of functional impairment by two weeks after birth resulting in death at 5 to 6 weeks postnatally. Pmn mice were therefore treated every second day (q2d) with vehicle (n=12) or 150 µg/kg PEG-IGF-I (n=13) s.c. from postnatal day 13 on, i.e. at a time when the disease just started. Using weekly assessment of muscle force of the fore limbs by analyzing grip strength, a clear effect of PEG-IGF-I was observed at postnatal day 45 where surviving pmn mice treated with PEG-IGF-I showed significantly higher performance compared to vehicle-treated animals (p<0.05, n=4-5, Fig. 5). Analysis of motor coordination by testing time spent on a rotarod revealed that PEG-IGF-I-treated pmn mice performed better than vehicle-treated mice, significant at postnatal day 38 (p<0.05, n=8-12, Fig. 6). Furthermore, histological analysis was peformed from pmn mice treated from postnatal day 13 on with vehicle or PEG-IGF-I (150 µg/kg s.c.) and perfused at postnatal day 34. Stereological counting of facial motoneurons revealed a significantly higher number of surviving motoneurons in the PEG-IGF-I treatment group (p<0.01, n=6-12, Fig. 7). Similarly, survival of motoneurons in the lumbar spinal cord was significantly increased (p<0.001, n=5-6, Fig. 8). Finally, analysis of the number of myelinated axons in the phrenic nerve revealed a significant higher number of myelinated axons in the proximal (p<0.05, n=4-5, Fig. 9) as well as the distal phrenic nerve (p<0.01, n=5-6, Fig. 10) when comparing vehicle- vs. PEG-IGF-I-treated pmn mice.

For testing of PEG-IGF-I in the most widely used model for familial ALS, SOD1(G93A) mice (low copy) were used. These mice develop first symptoms of disease by postnatal week 34-35 and death around 4-5 weeks later. SOD1(G93A) mice were therefore treated twice-a-week (q3.5d) with vehicle (n=6) or 150 µg/kg PEG-IGF-I (n=7) s.c. from postnatal week 34 on, i.e. at a time when the disease just started. For ensuring statistical power throughout the course of the experiment, LOCF (last observation carried forward) analysis was performed. This method (also used in clinical trials) maintains the last measurement of an animal before death for all subsequent time points. Analysis of body weight changes revealed that the drop of body weight in the early phase of the disease (around week 37) was significantly delayed in PEG-IGF-I-treated mice (p<0.05 for weeks 37, 38 and 39, n=6-7 LOCF, Fig. 11). Disease onset itself as measured by first signs of hindlimb weakness, abnormal gaits and difficulty to hold onto an inverted wire mesh was delayed on average by 4 weeks from postnatal week 38.5 to week 42.5 (p<0.05, n=6-7, Fig. 12). Using weekly assessment of muscle force of the fore limbs by analyzing grip strength, a significant protective effect of PEG-IGF-I was observed from postnatal week 35 on constantly until the death of all animals (p<0.05 for weeks 35, 38, 42 and 43, p<0.01 for weeks 36, 39, 40 and 41, n=6-7 LOCF, Fig. 13). Analysis of motor coordination by testing time spent on a rotarod revealed that PEG-IGF-I-treated SOD1(G93A) mice performed significantly better than vehicle-treated mice (p<0.05 for weeks 37, 38, 39 and 41, p<0.01 for weeks 40, 42 and 43, n=6-7 LOCF, Fig. 14).

Taken all *in vivo* data from pmn and SOD1(G93A) mice together, the studies have shown that PEG-IGF-I interferes with neuromuscular function in ALS models at all relevant targets and has the potential to act at every stage of disease. PEG-IGF-I was shown to preserve muscular force and function suggesting an anabolic effect on muscle, most probably by protecting the neuromuscular junction and connectivity. In addition to that, PEG-IGF-I was shown to rescue motor axons and motoneuron cell bodies in the spinal cord and facial nucleus suggesting a direct protective effect on motoneurons (Fig. 15). As these degenerations occur in a later stage of ALS, PEG-IGF-I can probably affect the course of disease at both early and later stages.

## Claims

1. Use of a polyethylene glycol-(PEG)ylated IGF-I variant for the manufacture of a pharmaceutical composition for the treatment, prevention and/or delay of a neuromuscular disorder selected from the group of neuropathy, muscular dystrophy and motor neuron disease (MND), wherein said polyethylene glycol-(PEG)ylated IGF-I variant is **characterized in that** it is derived from the wild-type human IGF-I amino acid sequence (SEQ ID NO: 1) and carries one or two amino acid alterations at amino acid positions 27, 65 and 68 so that one or two of amino acids at positions 27, 65 and 68 is/are a polar amino acid but not lysine and PEG is attached to at least one lysine residue.

2. Use as claimed in claim 1 wherein said PEGylated IGF-I variant is **characterized by** the following amino acid alterations of the wild-type human IGF-I amino acid sequence (SEQ ID NO: 1):
(a) K65R and K68R (SEQ ID NO: 2)
(b) K27R and K68R (SEQ ID NO: 3), or
(c) K27R and K65R (SEQ ID NO: 4).

3. Use as claimed in claim 1 or 2 wherein said PEGylated IGF-I variant is monoPEGylated at K68 and **characterized by** the following amino acid alterations of the wild-type human IGF-I amino acid sequence (SEQ ID NO: 1):
K27R and K65R (SEQ ID NO: 4).

4. Use as claimed in any of claims 1 to 3 wherein said PEG has an overall molecular weight of from 20 to 100 kDa.

5. Use as claimed in any of claims 1 to 4 wherein said PEGylated IGF-I variant is additionally PEGylated at the N-terminal amino acid.

6. Use as claimed in any of claims 1 to 5 wherein said PEGylated IGF-I variant is monoPEGylated at either K65 or K68, or is diPEGylated at K65 and K68.

7. Use as claimed in any of claims 1 to 6 wherein said PEGylated IGF-I variant is **characterized in that** up to three amino acids at the N-terminus are truncated.

8. Use as claimed in any of claims 1 to 7 wherein said PEGylated IGF-I variant is **characterized in that** the poly(ethylene glycol) group(s) is/are branched poly(ethylene glycol) group(s).

9. Use as claimed in any of claims 1 to 8 wherein said neuromuscular disorder is a motor neuron disease (MND).

10. Use as claimed in claim 9 wherein said MND is amyotrophic lateral sclerosis (ALS).

11. Use as claimed in claim 10 wherein the PEGylated IGF-I variant is used separately, sequentially or simultaneously in a combination combined with a second pharmacologically active compound.

12. Use as claimed in claim 11 wherein the second pharmacologically active compound of the combination is at least one neuroprotectant having an inhibitory effect on glutamate release or the effect of inactivation of voltage-dependent sodium channels or the ability to interfere with intracellular events that follow transmitter binding at excitatory amino acid receptors.

13. Use as claimed in claim 12 wherein the second pharmacologically active compound is riluzole.

14. Pharmaceutical composition comprising a PEGylated IGF-I variant according to any one
of claims 1 to 8 in a pharmaceutically acceptable form for use in a method for the treatment, prevention and/or delay of progression of a neuromuscular disorder selected from the group of neuropathy, muscular dystrophy and motor neuron disease (MND).

15. Pharmaceutical composition for use according to claim 14 wherein said neuromuscular disorder is a MND, preferably ALS.

## Patentansprüche

1. Verwendung einer Polyethylenglycol-(PEG)ylierten IGF-I-Variante für die Herstellung eines Arzneimittels zur Behandlung, Vorbeugung und/oder Verzögerung einer neuromuskulären Erkrankung, ausgewählt aus der Gruppe bestehend aus Neuropathie, Muskeldystrophie und Motoneuronenerkrankung (MND), wobei die Polyethylenglycol-(PEG)ylierte IGF-I-Variante **dadurch gekennzeichnet ist, dass** sie von der menschlichen Wildtyp-IGF-I-Aminosäuresequenz (SEQ ID NO:1) abstammt und mindestens ein oder zwei Aminosäureveränderungen an den Aminosäurepositionen 27, 65 und 68 trägt, so dass eine oder zwei der Aminosäuren an den Positionen 27, 65 und 68 eine polare Aminosäure, nicht aber Lysin, ist/sind, und das PEG an mindestens einen Lysinrest gebunden ist.

2. Verwendung nach Anspruch 1, wobei die PEGylierte IGF-I-Variante durch die folgenden Aminosäureveränderungen der menschlichen Wildtyp-IGF-I-Aminosäuresequenz (SEQ ID NO:1) gekennzeichnet ist:
(a) K65R und K68R (SEQ ID NO:2),
(b) K27R und K68R (SEQ ID NO:3), oder
(c) K27R und K65R (SEQ ID NO:4).

3. Verwendung nach Anspruch 1 oder 2, wobei die PEGylierte IGF-I-Variante an K68 monoPEGyliert ist und durch die folgenden Aminosäureveränderungen der menschlichen Wildtyp-IGF-I-Aminosäuresequenz (SEQ ID NO:1) gekennzeichnet ist:
K27R und K65R (SEQ ID NO:4).

4. Verwendung nach einem der Ansprüche 1 bis 3, wobei das PEG ein Gesamtmolekulargewicht von 20 bis 100 kDa hat.

5. Verwendung nach einem der Ansprüche 1 bis 4, wobei die PEGylierte IGF-I-Variante zusätzlich an der N-terminalen Aminosäure PEGyliert ist.

6. Verwendung nach einem der Ansprüche 1 bis 5, wobei die PEGylierte IGF-I-Variante entweder an K65 oder K68 monoPEGyliert oder an K65 und K68 diPEGyliert ist.

7. Verwendung nach einem der Ansprüche 1 bis 6, wobei die PEGylierte IGF-I-Variante **dadurch gekennzeichnet ist, dass** bis zu drei Aminosäuren am N-Terminus abgeschnitten sind.

8. Verwendung nach einem der Ansprüche 1 bis 7, wobei die PEGylierte IGF-I-Variante **dadurch gekennzeichnet ist, dass** die Poly(ethylenglycol)gruppe(n) (eine) verzweigte Poly(ethylenglycol)gruppe(n) ist/sind.

9. Verwendung nach einem der Ansprüche 1 bis 8, wobei die neuromuskuläre Erkrankung eine Motoneuronenerkrankung (MND) ist.

10. Verwendung nach Anspruch 9, wobei die MND amyotrophe Lateralsklerose (ALS) ist.

11. Verwendung nach Anspruch 10, wobei die PEGylierte IGF-I-Variante separat, sequenziell oder simultan in einer Kombination verbunden mit einer zweiten pharmakologisch aktiven Verbindung verwendet wird.

12. Verwendung nach Anspruch 11, wobei die zweite pharmakologisch aktive Verbindung der Kombination mindestens ein Neuroprotektivum ist, das eine inhibitorische Wirkung auf Glutamatfreisetzung oder die Wirkung zur Inaktivierung eines spannungsabhängigen Natriumkanals oder die Fähigkeit hat, intrazelluläre Ereignisse zu beeinträchtigen, die auf eine Transmitterbindung bei exzitatorisctten Aminosäurerezeptoren folgen.

13. Verbindung nach Anspruch 12, wobei die zweite pharmakologisch aktive Verbindung Riluzol ist.

14. Arzneimittel, umfassend eine PEGylierte IGF-I-Variante nach einem der Ansprüche 1 bis 8 in einer pharmazeutisch verträglichen Form zur Verwendung in einem Verfahren zur Behandlung, Vorbeugung und/oder Verzögerung des Verlaufs einer neuromuskulären Erkrankung, ausgewählt aus der Gruppe bestehend aus Neuropathie, Muskeldystrophie und Motoneuronenerkrankung (MND).

15. Arzneimittel zur Verwendung nach Anspruch 14, wobei die neuromuskuläre Erkrankung eine MND, bevorzugt ALS ist.

## Revendications

1. Utilisation d'une variante de polyéthylèneglycol-IGF-I (variante d'IGF-I pégylée) pour la fabrication d'une composition pharmaceutique destinée à traiter, prévenir et/ou retarder un trouble neuromusculaire choisi dans le groupe des neuropathies, des dystrophies musculaires et des maladies des neurones moteurs (MNM), où ladite variante d'IGF-I pégylée est **caractérisée en ce qu'**elle est dérivée de la séquence d'aminoacides d'IGF-I humaine de type sauvage (SEQ ID NO: 1) et qu'elle porte des modifications d'un ou deux aminoacides aux positions 27, 65 et 68 de manière qu'un ou deux des aminoacides aux positions 27, 65 et 68 soient des aminoacides polaires, mais non la lysine, et le PEG est lié à au moins un résidu de lysine.

2. Utilisation selon la revendication 1, où ladite variante d'IGF-I pégylée est **caractérisée par** les modifications d'aminoacides de la séquence d'aminoacides d'IGF-I humaine de type sauvage (SEQ ID NO: 1) suivantes:
(a) K65R et K68R (SEQ ID NO: 2),
b) K27R et K68R (SEQ ID NO: 3), ou
(c) K27R et K65R (SEQ ID NO: 4).

3. Utilisation selon la revendication 1 ou 2, où ladite variante d'IGF-I pégylée est monopégylée au niveau de K68 et **caractérisée par** les modifications d'aminoacides de la séquence d'aminoacides d'IGF-I humaine de type sauvage (SEQ ID NO: 1) suivantes:
K27R et K65R (SEQ ID NO: 4).

4. Utilisation selon l'une quelconque des revendications 1 à 3, où ledit PEG a une masse molaire globale de 20 à 100 kDa.

5. Utilisation selon l'une quelconque des revendications 1 à 4, où ladite variante d'IGF-I pégylée est en outre pégylée au niveau de l'aminoacide N-terminal.

6. Utilisation selon l'une quelconque des revendications 1 à 5, où ladite variante d'IGF-I pégylée est monopégylée au niveau de K65 ou de K68, ou est dipégylée au niveau de K65 et K68.

7. Utilisation selon l'une quelconque des revendications 1 à 6, où ladite variante d'IGF-I pégylée est **caractérisée en ce qu'**elle est tronquée d'au plus 3 aminoacides à l'extrémité N-terminale.

8. Utilisation selon l'une quelconque des revendications 1 à 7, où ladite variante d'IGF-I pégylée est **caractérisée en ce que** le ou les groupes de polyéthylèneglycol sont des groupes de polyéthylèneglycol ramifié.

9. Utilisation selon l'une quelconque des revendications 1 à 8, où ledit trouble neuromusculaire est une maladie des neurones moteurs (MNM).

10. Utilisation selon la revendication 9, où ladite MNM est une sclérose latérale amyotrophique (SLA).

11. Utilisation selon la revendication 10, où la variante d'IGF-I pégylée est utilisée séparément, successivement ou simultanément en combinaison avec un deuxième composé pharmacologiquement actif.

12. Utilisation selon la revendication 11, où le deuxième composé pharmacologiquement actif de la combinaison est au moins un neuroprotecteur ayant un effet inhibiteur sur la libération du glutamate ou l'effet d'inactivation des canaux sodiques voltage-dépendants ou la capacité de perturber des événements intracellulaires qui suivent la liaison d'un transmetteur aux récepteurs d'aminoacides excitateurs.

13. Utilisation selon la revendication 12, où le deuxième composé pharmacologiquement actif est le riluzole.

14. Composition pharmaceutique comprenant une variante d'IGF-I pégylée selon l'une quelconque des revendications 1 à 8 sous une forme pharmaceutiquement acceptable à utiliser dans un procédé de traitement, de prévention ou de retardement de l'évolution d'un trouble neuromusculaire choisi dans le groupe des neuropathies, des dystrophies musculaires et des maladies des neurones moteurs (MNM).

15. Composition pharmaceutique à utiliser selon la revendication 14, où le trouble neuromusculaire est une MNM, de préférence la SLA.
